# EUROPEAN PATENT SPECIFICATION

(11) **EP 2 917 178 B1**
(45) Date of publication and mention of the grant of the patent: **09.11.2016**
(21) Application number: 13814237.7
(22) Date of filing: 30.10.2013
(51) Int. Cl.: C07C 323/12

(54) **SULPHUR CONTAINING HIGH REFRACTIVE INDEX MONOMER**
SCHWEFELHALTIGES MONOMER MIT HOHEM BRECHUNGSINDEX
MONOMÈRE À INDICE DE RÉFRACTION ÉLEVÉ CONTENANT DU SOUFRE

(30) Priority: 07.11.2012 IN DE34402012
(43) Date of publication of application: 16.09.2015
(73) Proprietor: Council of Scientific & Industrial Research, New Delhi 110 001 (IN)
(72) Inventor: PONRATHNAM, Surendra, Pune Maharashtra 411 008 (IN); GHORPADE, Ravindra Vasant, Pune Maharashtra 411 008 (IN); CHAVAN, Nayaku Nivrati, Pune Maharashtra 411 008 (IN); RAJDEO, Kishor Sudam, Pune Maharashtra 411 008 (IN); BHONGALE, Sunil Sitaram, Pune Maharashtra 411 008 (IN)
(74) Representative: Nuss, Laurent
(86) International application number: PCT/IN2013/000659
(87) International publication number: WO 2014/072995

(56) References cited:
- US-A1- 2008 200 582

## Description

### FIELD OF THE INVENTION

Present invention relates to sulphur containing high refractive index monomer, 2-phenyl-2-(phenylthio)ethyl acrylate. Particularly, present invention relates to process for the preparation of high refractive index monomer. More particularly, present invention relates to high refractive index monomer find applications in plastic materials wherein the high refractive index property can be advantageously used to make light weight materials with high transparency.

### BACKGROUND AND PRIOR ART OF THE INVENTION

Amongst several optical properties, refractive index is one of the most important criteria for selection of materials suitable for application in optics. One of the possible candidates for this purpose is plastic materials. Other than the essential properties like light transmission, transparency and refractive index, plastics are also known for their light weight. High refractive index is the important property required for a transparent synthetic resin as optical material. Transparent synthetic resin having high refractive index when used as optical products can be rendered thinner than material having low refractive index to give the same focal distance. The use of thin lenses contributes to reducing the volume of space occupied by lenses in optical assemblies, which can advantageously make an optical apparatus light weight and small sized.

Transparent composite materials are becoming increasingly important in applications ranging from protective coatings to optical communications. Optical materials are useful to control the flow and intensity of light. Examples of useful optical products include optical light fibers, light tubes, optical films including totally internal reflecting films, retroreflective sheeting, ophthalmic lenses. These monomers can be used in compositions to make light management films advantageously provide a cured composition having high refractive index. Even for applications such as liquid crystal displays, adhesives, magnifying lenses, binoculars, and telescopes requires monomers having high refractive index. High refractive index polymers have a wide variety of applications in dentistry, optical eyewear, holography, and microelectronics.

Optical products can be prepared from high index of refraction materials, including monomers such as high index of refraction (meth) acrylate monomers, aromatic based monomers, and other such high index of refraction monomers that are known. The monomers can be cured or polymerized to take the form of a product capable of modifying or controlling the flow of light.

Current high RI plastics include polyurethanes, polyesters, epoxy and episulfide resins. Most of the high RI plastics use thiourethane and episulfide chemistries with highly polarizable chemical moieties such as aromatics and sulfur. However, optical articles produced from these materials suffer from after-cure yellowing and strong odors released during lens processing. In addition, these monomers have inherently long production cycles due to prolonged curing times needed for maintaining optical homogeneity. There is, therefore, a need for monomers which offer fast cure, high RI, low color, and low odor when cured or upon cutting and grinding, while maintaining optical homogeneity.

The general approach to increase the refractive indices of polymers is the introduction of substituent with high-molar refraction, low-molar volume, or high density, according to the Lorentz-Lorenz equation. Among the various substituents, the sulfur (S) atom is one of the most effective candidates because of its high atomic refraction. Recently, sulfur-containing polymers with thioether linkages have been developed for optical applications.

There is ample literature available in this area as discussed below.

There are many publications related to preparation of aromatic alcohol and high refractive index monomers for example, J. Org. Chem., Vol. 68, No. 21, 2003; Synlett. No. 15, pp 2292-2296, 2003; Polymer Journal 42, 249-255, 2010; Journal of Macromolecular Science, Part A, 36: 9, 1271-1288, 1999; and Journal of Macromolecular Science, Part A, 37: 3, 239 - 257,2000.

There number of patents/patent publications pertaining to the preparation and applications of sulfur containing high refractive index monomers and their applications in optical articles as listed below.

US 6528601 discloses polymerizable sulfur-containing (meth)acrylate, having good refractive index, Abbe's number and light resistance and is suitable for the production of a transparent resin which rarely smells at the time of molding and is further with excellent in storage stability.

WO2008/101806 discloses high refractive index methaacrylic monomers, compositions and use of the same.

Polymerizable composition comprising at least one monomer of mono(thio) (meth)acrylate or di(meth)acrylate type bearing a 5- to 8-membered heterocycle consisting of C, H and S atoms and containing 2 or 3 endocyclic sulphur atoms, the heterocycle optionally being fused to a substituted or unsubstituted C5-C8 aromatic or C6-C7 polycyclic ring are disclosed in US 6472488.

WO2007094665 describes intraocular lenses essentially free from glistening and a method for manufacturing an intraocular lens in a closed mould. The process includes polymerization of an acrylic monomer composition containing a single high refractive index monomer by employing an initiator that is activated by light having a wavelength of 390 nm or more.

US2008/0200582 describes process for the preparation of 2-Phenyl-2-phenylthioethyl methacrylate of formula given below:

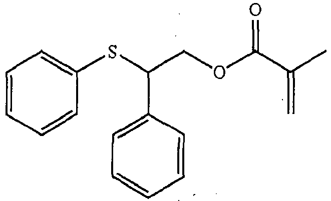

### 2-Phenyl-2-phenylthioethyl methacrylate

Referctive index of the said monomer is 1.576(Tg=68°C). Monomer of the present invention exhibit 1.584 referactive index (Tg=25°C). There is major difference in glass transition temperatures (Tg) of homopolymers of these two monomers, which is very important for intraocular lens (IOL)application. For IO1 applications, polymeric material should have elastomeric properties to obtain a foldable IOL and avoid plastic deformation. Low Tg (25 °C) of Poly(2-PHENYL-2-(PHENYLTHIO) ACRYLATE) of formula I provide flexibility and elastomeric property to copolymers of IOL material.

Therefore, there is a need in the art to provide an alternate acrylate monomer with high refractive index compared to known ones. The acrylate monomer of the invention finds applications in in plastic materials wherein the high refractive index property can be advantageously used to make light weight materials with high transparency.

### OBJECTS OF THE INVENTION

Main object of the present invention is to provide sulphur containing high refractive index monomer namely 2-phenyl-2-(phenylthio)ethyl acrylate of formula I.

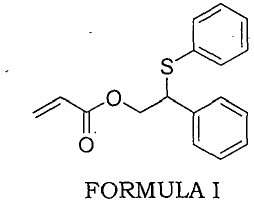

Another object of the present invention is to provide process for the preparation of high refractive index monomer.

Yet another object of the present invention is to provide high refractive index monomer find applications in plastic materials wherein the high refractive index property can be advantageously used to make light weight materials with high transparency.

### BRIEF DESCRIPTION OF DRAWINGS

- Fig 1: shows 1H NMR spectra of the monomer of formula I.
- Fig 2: shows 13C NMR spectra of the monomer of formula I.
- Fig 3: shows 13C NMR-DEPT spectra of the monomer of formula I.
- Fig 4: depicts IR Spectra of the monomer of formula I.

### SUMMARY OF THE INVENTION

Accordingly, present invention provides compound of formula I

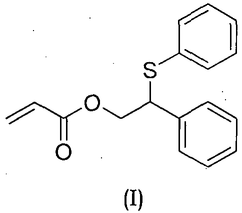

In an embodiment of the present invention, refractive index of the said compound is 1.584.

In another embodiment, present invention provides a process for the preparation of compound of formula 1 the said process comprising the steps of:
i. adding 2-phenyl-2-(phenylthio)ethanol of the formula (III) in a solvent followed by cooling in an ice bath under nitrogen; 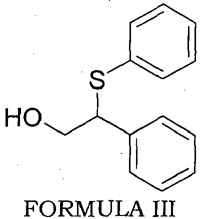
ii. optionally adding dehydrohalogenating agent in the solution as obtained in step (i) under stirring;
iii. adding acrylol chloride of the formula (II) in a solvent to obtain a solution; 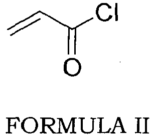
iv. adding the solution as obtained in step (iii) in solution as obtained in step (ii) with stirring at temperature in the range of 0 to 4°C for period in the range of 1 to 2 hrs;
v. stirring the mixture at temperature in the range of 22 to 30°C for period in the range of 2 to 24 hrs followed by adding methanol at temperature in the range of 4 to 20 °C to obtain crude product;
vi. washing, drying and purifying the crude product as obtained in step (v) by column chromatography to obtain 85 to 97% 2-phenyl-2-(phenylthio)ethyl acrylate of the formula (I) based on the charged 2-phenyl-2-(phenylthio)ethanol of formula III.

In yet another embodiment of the present invention, solvent used is selected from the group consisting of water, esters, ketones, ethers, alcohols, aromatic hydrocarbons, haloalkanes, heterocyclic compounds.

In yet another embodiment of the present invention, solvent used is selected from the group consisting of dichloromethane, tetrahydrofuran, toluene, ethyl methyl ketone, chloroform, ethyl acetate or petroleum ether or combination thereof.

In yet another embodiment of the present invention, solvent used is 1 to 10 times preferably 1 to 5 times by weight with respect to the total amount of the acryloyl chloride and the 2-phenyl-2-(phenylthio)ethanol.

In yet another embodiment of the present invention, dehydrohalogenating agent used is selected from the group consisting of triethylamine, trimethylamine, pyridine, sodium carbonate and potassium carbonate.

In yet another embodiment of the present invention, dehydrohalogenating agent used is 1 to 3 times by mol, preferably 1 to 1.5 times by mol with respect the 2-phenyl-2-(phenylthio)ethanol.

In yet another embodiment of the present invention, acryloyl chloride of the formula (II) is used in a ratio of from about 1 to about 3 mol per mol of the 2-phenyl-2-(phenylthio)ethanol of the formula (III).

In yet another embodiment of the present invention, said compound is useful for making thinner and light weight optical materials, intraocular lenses.

### DETAILED DESCRIPTION OF INVENTION

Present invention provides high refractive index monomer namely 2-phenyl-2-(phenylthio)ethyl acrylate of formula I.

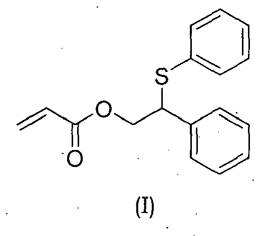

Present invention further provides a process for the preparation of high refractive index monomer namely 2-phenyl-2-(phenylthio)ethyl acrylate of formula I and the said process comprising: reacting acrylol chloride of the formula (II) with a 2-phenyl-2-(phenylthio)ethanol of the formula (III) at a temperature range of 0 to 50°C to obtain the 2-phenyl-2-(phenylthio)ethyl acrylate of the formula (I).

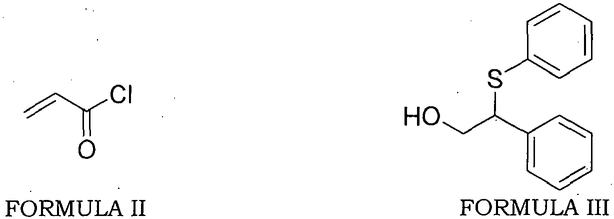

As described above, the process for the preparation of novel monomer with high refractive index comprises preparation of aromatic alcohol of formula III by a process of known reaction followed by reaction of this alcohol with acryloyl chloride of formula II to get the product of interest.

In the first step of the process of the present invention, an aromatic nucleophile thiophenol is reacted with styrene oxide in the presence of Zinc chloride in aqueous medium to form 2-phenyl-2-(phenylthio)ethanol of the formula (III).

In the second step of the process of the present invention, the condensation reaction of the acryloyl chloride of the formula (II) with the 2-phenyl-2-(phenylthio)ethanol of the formula (III) to produce the 2-phenyl-2-(phenylthio)ethyl acrylate of the formula (I) can be conducted. For example, the acryloyl chloride of the formula (II) and the 2-phenyl-2-(phenylthio)ethanol of the formula (III) may be reacted in an appropriate solvent. As a solvent used in this reaction, organic polar solvents are advantageous. Examples thereof include ketones such as methyl ethyl ketone and ethers such as tetrahydrofuran, and chlorinated solvents such as dichloromethane, chloroform. Amount of the solvent preferably ranges approximately from 1 to 10 times by weight with respect to the total amount of the acryloyl chloride and the 2-phenyl-2-(phenylthio)ethanol. An amount as much as approximately from 1 to 5 times by weight is more preferably used from considerations of volume efficiency.

In this reaction, it is advantageous that the reaction is carried out in the presence of a dehydrohalogenating agent because a hydrogen chloride is formed by condensation. Examples of the dehydrohalogenating agent usable in the above case include amines such as triethylamine and pyridine, and inorganic bases such as sodium carbonate and potassium carbonate. In particular, an organic amine is preferable. When a dehydrohalogenating agent is used, for example, following methods can be adopted:
a method wherein the 2-phenyl-2-(phenylthio)ethanol of the formula (III) and the dehydrohalogenating agent are added in an adequate solvent and then the acryloyl chloride of the formula (II) are gradually added thereto; and a method wherein the acryloyl chloride of the formula (II) and the 2-phenyl-2-(phenylthio)ethanol of the formula (III) are dissolved in an adequate solvent and the dehydrohalogenating agent is gradually added thereto. When the dehydrohalogenating agent is used, the amount thereof is generally approximately from 1 to 3 times by mol, preferably approximately from 1 to 1.5 times by mol with respect the 2-phenyl-2-(phenylthio)ethanol.

In this reaction, a molar ratio of the acryloyl chloride of the formula (II) to the 2-phenyl-2-(phenylthio)ethanol of the formula (III) is important to make the reaction proceed smoothly. In general, it is desirable that the acryloyl chloride of the formula (II) is used in a ratio of from about 1 to about 3 mol per mol of the 2-phenyl-2-(phenylthio)ethanol of the formula (III), although preferably molar ratio varies depending upon the type of the dehydrohalogenating agent. When an organic amine is used as the dehydrohalogenating agent, the acryloyl chloride of the formula (II) is preferably as much as approximately from 1.1 to 2.5 times by mol with respect to the 2-phenyl-2-(phenylthio)ethanol of the formula (III). When an inorganic base is used as the dehydrohalogenating agent, the acryloyl halide is preferably as much as approximately from 2 to 3 times by mol with respect to the 2-phenyl-2-(phenylthio)ethanol.

This reaction can proceed at any temperature between low temperatures of about -10 °C to a high temperature of about the boiling point of the reaction solvent. However, it is economical to conduct the reaction at temperatures ranging from about 0 to 50°C preferably at 0°C to 35°C. Further, the reaction can be conducted under atmospheric pressure, and the reaction time is approximately from 2 to 24 hours. When the condensation reaction has completed, the unreacted acryloyl chloride is converted into an ester and deactivated by addition of alcohol, if necessary. Further, washing with water and an aqueous sodium chloride solution of the reaction mass is conducted.

Typically in the reaction to prepare high refractive index monomers, the product contains impurities. The removal of color from the prepared monomers may be carried out by column chromatography using silica gel as absorbent and petroleum ether: ethyl acetate mixture as a solvent.

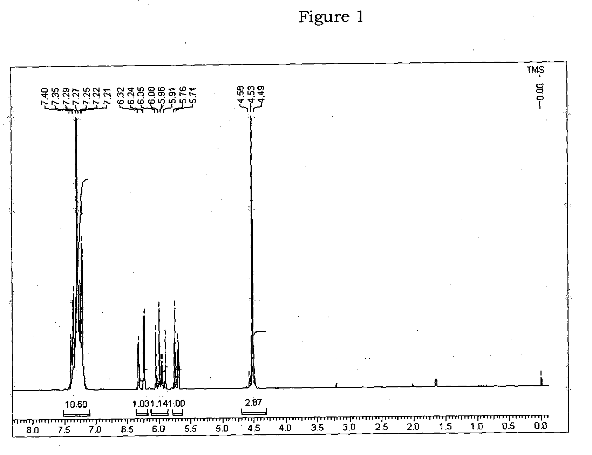

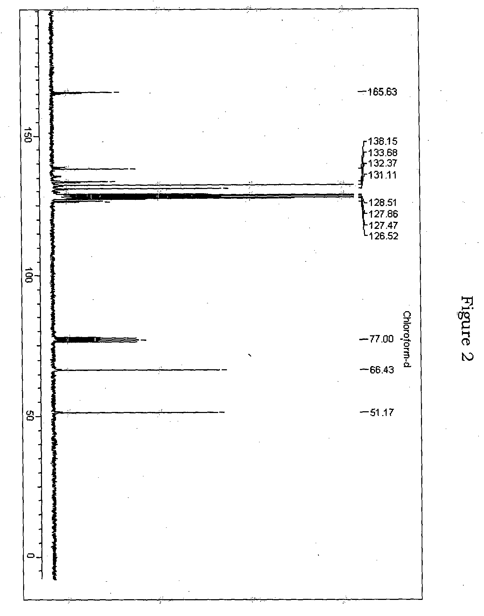

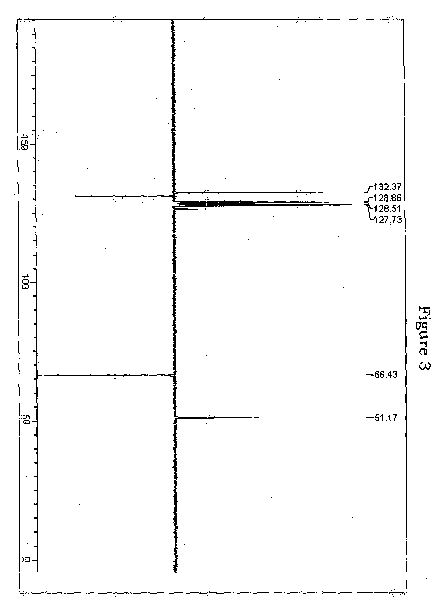

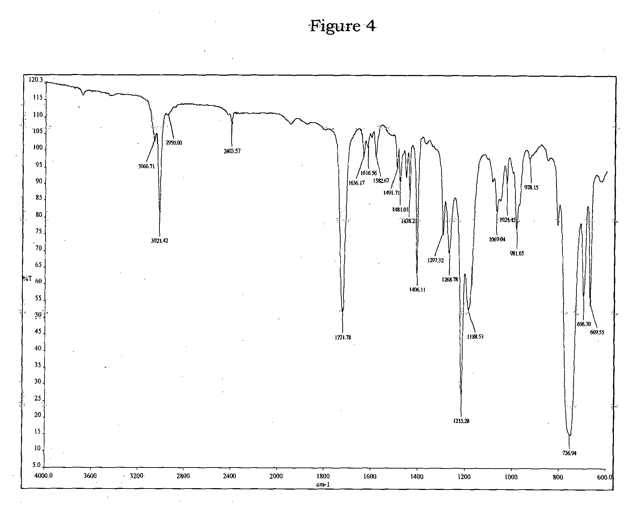

The monomer of the present invention is subjected to spectral evaluation using ¹H NMR, ¹³C NMR, ¹³C NMR-DEPT and IR Spectra as given in figure 1 to 4.

This monomer can be polymerized to take the form of an optical products having high refractive index. Optical products made from this monomer having high refractive index can be thinner and provide same refractive power as product made from a material having a relatively low refractive index. In particular the polymeric material prepared from the novel monomer can be used for making intraocular lenses that are thinner than the conventionally used ones offering advantages of reduced volume of space and light weight materials.

### EXAMPLES

The following examples are given by way of illustration and therefore should not be construed to limit the scope of present invention.

### EXAMPLE 1

### Synthesis of 2-phenyl-2-(phenylthio)ethanol (FORMULA III)

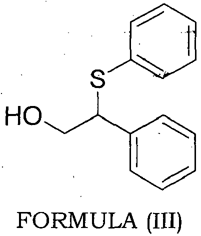

In a three necked round bottom flask thermostated at 30°C and equipped with a magnetic stirrer, dropping funnel and a pH electrode, thiophenol (0.15 mL, 1.5 mmol) and 0.5 mL of a 0.1 M aq solution of ZnCl₂ (0.05 mmol) were added to 0.7 mL of water. The resulting pH was adjusted to 4.0 by adding 0.05 mL of aq 5 M NaOH. The styrene oxide (1.0 mmol) was then added under stirring. After the reaction aq. 5 M NaOH was added to the resulting mixture to achieve a pH of 9.0 and the reaction mixture was extracted with ethyl acetate. Further organic layer dried over anhydrous sodium sulphate and ethyl acetate was removed under reduced pressure. Viscous light yellow coloured liquid product was obtained.

### EXAMPLE 2

### Synthesis of 2-phenyl-2-(phenylthio)ethyl acrylate (Formula I)

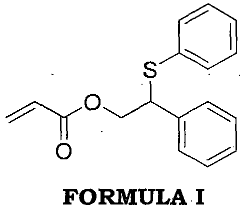

In a three necked flask equipped with a mechanical stirrer, nitrogen inlet, and guard tube charged 30.0 g (0.13 mol) of 2-phenyl-2-(phenylthio)ethanol, 100 ml of dry distilled dichloromethane. The reaction flask was cooled in an ice bath under nitrogen. 17.13 g (0.17 mol) triethylamine was added by dropping funnel under stirring. Acryloyl chloride 15.56 g (0.17 mol) in 100 ml dry dichloromethane was taken in dropping funnel, added slowly, drop wise with stirring by maintaining temperature at 4 °C throughout the addition. After the addition reaction mixture was kept stirring at same temperature for 2 hours. Reaction further proceeds at 25 °C for 24 hours. Reaction was monitored by TLC (30 % DCM in hexane). After completion of reaction 3.20 g (0.1 mol) of methanol was added dropwise to deactivate the unreacted acryloyl chloride while keeping the temperature below 20°C. Then, 200 mL of water was added thereto to wash the mixture, followed by separation of the mixture into layers. Thereafter organic layer was washed with 4 % NaOH solution, brine solution and distilled water. Organic layer was dried over sodium sulphate and dichloromethane was removed under reduced pressure to obtain liquid 2-phenyl-2-(phenylthio) ethyl acrylate. Crude product was purified by column chromatography (silica gel) using petroleum ether: ethyl acetate (95:05) mixture to yield 34.78 g of colorless transparent liquid. The yield was 93% based on the charged 2-phenyl-2-(phenylthio)ethanol. Refractive index of monomer is 1.584.

### EXAMPLE 3

### Synthesis of 2-phenyl-2-(phenylthio)ethyl acrylate (Formula I)

In a three necked flask equipped with a mechanical stirrer, nitrogen inlet, and guard tube charged 16.30 g (0.07 mol) of 2-phenyl-2-(phenylthio)ethanol, 50 ml of dry distilled ethyl methyl ketone. The reaction flask was cooled in an ice bath under nitrogen. 9.31 g (0.09 mol) triethylamine was added by dropping funnel under stirring. Acryloyl chloride 8.42 g (0.09 mol) in 30 ml dry ethyl methyl ketone was taken in dropping funnel, added slowly, drop wise with stirring by maintaining temperature at 4 °C throughout the addition. After the addition reaction mixture was kept stirring at same temperature for 2 hours. Reaction further proceeds at room temperature i.e.23°C for 24 hours. Reaction was monitored by TLC (30 % DCM in hexane). After completion of reaction 1.7 g (0.05 mol) of methanol was added dropwise to deactivate the unreacted acryloyl chloride while keeping the temperature below 20 °C. Then, 100 mL of water was added thereto to wash the mixture, followed by separation of the mixture into layers. Thereafter organic layer was washed with 4 % NaOH solution, brine solution and distilled water. Organic layer was dried over sodium sulphate and solvent was removed under reduced pressure to obtain liquid 2-phenyl-2-(phenylthio)ethyl acrylate. Crude product was purified by column chromatography (silica gel) using petroleum ether: ethyl acetate (95:05) mixture to yield 17.11 g of colorless transparent liquid. The yield was 85% based on the charged 2-phenyl-2-(phenylthio)ethanol. Refractive index of monomer is 1.584.

### EXAMPLE 4

### Synthesis of 2-phenyl-2-(phenylthio)ethyl acrylate (Formula I)

In a three necked flask equipped with a mechanical stirrer, nitrogen inlet, and guard tube charged 12.30 g (0.043 mol) of 2-phenyl-2-(phenylthio)ethanol, 30 ml of dry distilled tetrahydrofuran. The reaction flask was cooled in an ice bath under nitrogen. 5.69 g (0.056 mol) triethylamine was added by dropping funnel under stirring. Acryloyl chloride 5.14 g (0.056 mol) in 20 ml dry tetrahydrofuran was taken in dropping funnel, added slowly, drop wise with stirring by maintaining temperature at 4°C throughout the addition. After the addition reaction mixture was kept stirring at same temperature for 2 hours. Reaction further proceeds at 25 °C for 24 hours. Reaction was monitored by TLC (30 % DCM in hexane). After completion of reaction 1.60g (0.05 mol) of methanol was added dropwise to deactivate the unreacted acryloyl chloride while keeping the temperature below 20°C. Then, 50 mL of water was added thereto to wash the mixture, followed by separation of the mixture into layers. Thereafter organic layer washed with 4 % NaOH solution, brine solution and distilled water. Organic layer was dried over sodium sulphate and tetrahydrofuran was removed under reduced pressure to obtain liquid 2-phenyl-2-(phenylthio)ethyl acrylate. Crude product was purified by column chromatography (silica gel) using petroleum ether: ethyl acetate (95:05) mixture to yield 13.98 g of colorless transparent liquid. The yield was 92% based on the charged 2-phenyl-2-(phenylthio)ethanol. Refractive index of monomer is 1.584.

### EXAMPLE 5

### Synthesis of 2-phenyl-2-(phenylthio)ethyl acrylate (Formula I)

In a three necked flask equipped with a mechanical stirrer, nitrogen inlet, and guard tube charged 12.30 g (0.043 mol) of 2-phenyl-2-(phenylthio)ethanol, 20 ml of dry distilled dichloromethane. The reaction flask was cooled in an ice bath under nitrogen. 4.79g (0.06 mol) pyridine was added by dropping funnel under stirring. Acryloyl chloride 5.14 g (0.056 mol) in 20 ml dry dichloromethane was taken in dropping funnel, added slowly, drop wise with stirring by maintaining temperature at 4 °C throughout the addition. After the addition reaction mixture was kept stirring at same temperature for 2 hours. Reaction further proceeds at room temperature i.e. 25°C for 8 hours. Reaction was monitored by TLC (30 % DCM in hexane). After completion of reaction 1.50 g of methanol was added dropwise to deactivate the unreacted acryloyl chloride while keeping the temperature below 20 °C. Then, 50 mL of water was added thereto to wash the mixture, followed by separation of the mixture into layers. Thereafter organic layer was washed with 4 % NaOH solution followed by brine solution and distilled water. Organic layer was dried over sodium sulphate and dichloromethane was removed under reduced pressure to obtain liquid 2-phenyl-2-(phenylthio) ethyl acrylate. Crude product was purified by column chromatography (silica gel) using petroleum ether: ethyl acetate (95:05) mixture to yield 14.26 g of colorless transparent liquid. The yield was 94% based on the charged 2-phenyl-2-(phenylthio)ethanol. Refractive index of monomer is 1.584.

### ADVANTAGES OF THE INVENTION

- The novel monomer prepared according to the process of the invention and as described in examples 2 to 5 lead to aromatic based hydrophobic monomers with high refractive index (η = 1.58).
- The monomers prepared as per the process of invention obviate any derivatization or treatment to polymerization to prepare materials with high refractive index and excellent transparency properties.
- The polymers prepared from monomers as per the process of invention exhibits high transparency and can be used for making optical articles such as intraocular lenses. High refractive index optical materials can dramatically reduce optics profile and make optical article lighter and thinner

## Claims

1. Compound of formula I

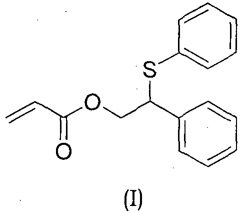

2. The compound as claimed in claim 1, wherein refractive index of the said compound is 1.584.

3. A process for the preparation of compound of formula 1 as claimed in claim 1 and the said process comprising the steps of:
i. adding 2-phenyl-2-(phenylthio)ethanol of the formula (III) in a solvent followed by cooling in an ice bath under nitrogen; 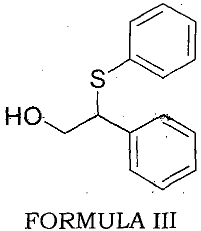
ii. optionally adding dehydrohalogenating agent in the solution as obtained in step (i) under stirring;
iii. adding acrylol chloride of the formula (II) in a solvent to obtain a solution; 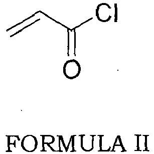
iv. adding the solution as obtained in step (iii) in solution as obtained in step (ii) with stirring at temperature in the range of 0 to 4°C for period in the range of 1 to 2 hrs;
v. stirring the mixture at temperature in the range of 22 to 30°C for period in the range of 2 to 24 hrs followed by adding methanol at temperature in the range of 4 to 20 °C to obtain crude product;
vi. washing, drying and purifying the crude product as obtained in step (v) by column chromatography to obtain 85 to 97% 2-phenyl-2-(phenylthio)ethyl acrylate of the formula (I) based on the charged 2-phenyl-2-(phenylthio)ethanol of formula III.

4. The process of claim 3, wherein in step (i) and step (iii) the solvent used is selected from the group consisting of water, esters, ketones, ethers, alcohols, aromatic hydrocarbons, haloalkanes, heterocyclic compounds.

5. The process as claimed in claim 4, wherein solvent used is selected from the group consisting of dichloromethane, tetrahydrofuran, toluene, ethyl methyl ketone, chloroform, ethyl acetate or petroleum ether or combination thereof.

6. The process of claim 3, wherein in step (i) the solvent used is 1 to 10 times preferably 1 to 5 times by weight with respect to the total amount of the acryloyl chloride and the 2-phenyl-2-(phenylthio) ethanol.

7. The process as claimed in claim 3, wherein dehydrohalogenating agent used is selected from the group consisting of triethylamine, trimethylamine, pyridine, sodium carbonate and potassium carbonate.

8. The process as claimed in claim 3, wherein dehydrohalogenating agent used is 1 to 3 times by mol, preferably 1 to 1.5 times by mol with respect the 2-phenyl-2-(phenylthio)ethanol.

9. The process as claimed in claim 3, wherein acryloyl chloride of the formula (II) is used in a ratio of from about 1 to about 3 mol per mol of the 2-phenyl-2-(phenylthio)ethanol of the formula (III).

10. The compound of formula I as claimed in claim 1, wherein said compound is useful for making thinner and light weight optical materials, intraocular lenses.

## Patentansprüche

1. Verbindung der Formel I

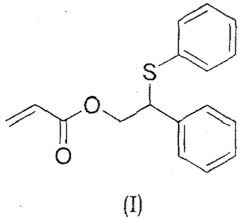

2. Verbindung nach Patentanspruch 1, wobei der Brechungsindex der genannten Verbindung 1,584 beträgt.

3. Verfahren zur Herstellung der Verbindung der Formel I nach Patentanspruch 1, wobei das genannte Verfahren die folgenden Schritte umfasst:
i. Zusatz von 2-Phenyl-2-(Phenylthio)ethanol nach Formel (III) zu einem Lösungsmittel, gefolgt von Abkühlung im Eisbad unter Stickstoff, 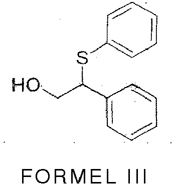
ii. optional Zusatz eines Halogenwasserstoff abspaltenden Mittels zur in Schritt (i) erhaltenen Lösung unter Rühren,
iii. Zusatz von Acryloylchlorid nach Formel (II) zu einem Lösungsmittel, um eine Lösung zu erhalten, 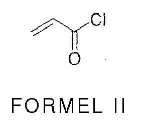
iv. Zusatz der in Schritt (iii) erhaltenen Lösung zur in Schritt (ii) erhaltenen Lösung unter Rühren bei einer Temperatur im Bereich von 0 bis 4°C für eine Dauer im Bereich von 1 bis 2 Stunden,
v. Rühren des Gemisches bei einer Temperatur im Bereich von 22 bis 30°C für eine Dauer im Bereich von 2 bis 24 Stunden, gefolgt von Zusatz von Methanol bei einer Temperatur im Bereich von 4 bis 20°C, um das Rohprodukt zu erhalten,
vi. Waschen, Trocknen und Reinigen des in Schritt (v) erhaltenen Rohproduktes durch Säulenchromatographie, um 85 bis 97% 2-Phenyl-2-(phenylthio)ethylacrylat der Formel (I), bezogen auf die Menge des eingebrachten 2-Phenyl-2-(phenylthio)ethanols der Formel (III), zu erhalten.

4. Verfahren nach Patentanspruch 3, wobei das in den Schritten (i) und (iii) verwendete Lösungsmittel aus der Gruppe, bestehend aus Wasser, Estern, Ketonen, Äthern, Alkoholen, aromatischen Kohlenwasserstoffen, Halogenalkanen, heterozyklischen Verbindungen, ausgewählt wird.

5. Verfahren nach Patentanspruch 4, wobei das verwendete Lösungsmittel aus der Gruppe, bestehend aus Dichlormethan, Tetrahydrofuran, Toluol, Ethylmethylketon, Chloroform, Ethylazetat oder Petroläther oder Gemischen davon, ausgewählt wird.

6. Verfahren nach Patentanspruch 3, wobei das in Schritt (i) verwendete Lösungsmittel das 1- bis 10-fache, vorzugsweise das 1- bis 5-fache des Gewichts der gesamten Menge an Acryloylchlorid und 2-Phenyl-2-(phenylthio)ethanol beträgt.

7. Verfahren nach Patentanspruch 3, in dem das verwendete Halogenwasserstoff abspaltende Mittel aus der Gruppe, bestehend aus Triethylamin, Trimethylamin, Pyridin, Natriumkarbonat und Kaliumkarbonat, ausgewählt wird.

8. Verfahren nach Patentanspruch 3, in dem das 1- bis 3-fache, vorzugsweise das 1- bis 1,5-fache in Mol des 2-Phenyl-2-(phenylthio)ethanols an Halogenwasserstoff abspaltendem Mittel verwendet wird.

9. Verfahren nach Patentanspruch 3, in dem das Acryloylchlorid der Formel (II) in einem Verhältnis von 1 bis 3 Mol je Mol 2-Phenyl-2-(phenylthio)ethanol der Formel (III) verwendet wird.

10. Verbindung der Formel I nach Patentanspruch 1, wobei die genannte Verbindung dafür brauchbar ist, dünneres und leichtes optisches Material, Intraokularlinsen herzustellen.

## Revendications

1. Composé de formule I

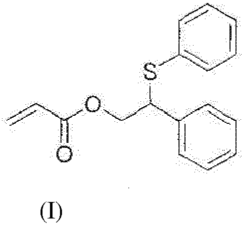

2. Composé selon la revendication 1, dans lequel l'indice de réfraction dudit composé est de 1,584.

3. Procédé pour la préparation d'un composé de formule 1 selon la revendication 1 et ledit procédé comprenant les étapes consistant à :
i. ajouter du 2-phényl-2-(phénylthio)éthanol de la formule (III) dans un solvant suivi par un refroidissement dans un bain de glace sous azote ; 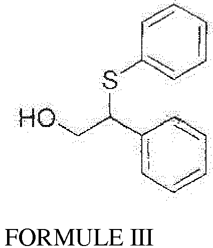
ii. optionnellement ajouter un agent de déshydrohalogénation dans la solution telle qu'obtenue à l'étape (i) sous agitation ;
iii. ajouter du chlorure d'acryloyle de la formule (II) dans un solvant pour obtenir une solution ; 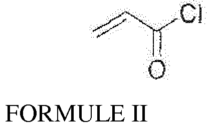
iv. ajouter la solution telle qu'obtenue à l'étape (iii) dans une solution telle qu'obtenue à l'étape (ii) avec une agitation à une température dans la plage de 0 à 4 °C pendant une durée dans la plage de 1 à 2 heures ;
v. agiter le mélange à une température dans la plage de 22 à 30 °C pendant une durée dans la plage de 2 à 24 heures suivi par l'ajout de méthanol à une température dans la plage de 4 à 20 °C pour obtenir un produit brut ;
vi. laver, sécher et purifier le produit brut tel qu'obtenu à l'étape (v) par chromatographie en colonne pour obtenir de 85 à 97 % de 2-phényl-2-(phénylthio)éthyl acrylate de la formule (I) à partir du 2-phényl-2-(phénylthio)éthanol chargé de la formule III.

4. Procédé selon la revendication 3, dans lequel dans l'étape (i) et l'étape (iii) le solvant utilisé est sélectionné à partir du groupe constitué de l'eau, des esters, des cétones, des éthers, des alcools, des hydrocarbures aromatiques, des haloalcanes, des composés hétérocycliques.

5. Procédé selon la revendication 4, dans lequel le solvant utilisé est sélectionné à partir du groupe constitué du dichlorométhane, du tetrahydrofurane, du toluène, de l'éthyl méthyl cétone, du chloroforme, de l'acétate d'éthyle ou des combinaisons de ceux-ci.

6. Procédé selon la revendication 3, dans lequel dans l'étape (i) le solvant utilisé correspond à 1 à 10 fois de préférence de 1 à 5 fois en poids par rapport à la quantité totale de chlorure d'acryloyle et de 2-phényl-2-(phénylthio)éthanol.

7. Procédé selon la revendication 3, dans lequel l'agent de déshydrohalogénation est sélectionné à partir du groupe constitué de la triéthylamine, de la triméthylamine, de la pyridine, du carbonate de sodium et du carbonate de potassium.

8. Procédé selon la revendication 3, dans lequel l'agent de déshydrohalogénation correspond à 1 à 3 fois en mole, de préférence de 1 à 1,5 fois en mole par rapport au 2-phényl-2-(phénylthio)éthanol.

9. Procédé selon la revendication 3, dans lequel le chlorure d'acryloyle de la formule (II) est utilisé suivant un rapport entre environ 1 et environ 3 moles par mole de 2-phényl-2-(phénylthio)éthanol de la formule (III).

10. Composé de formule I selon la revendication 1, où ledit composé est utile pour réaliser des matériaux optiques plus minces et plus légers, lentilles intraoculaires.
